# EUROPÄISCHE PATENTSCHRIFT

(11) **EP 1 257 364 B1**
(45) Veröffentlichungstag und Bekanntmachung des Hinweises auf die Patenterteilung: **23.10.2013**
(21) Anmeldenummer: 01907539.9
(22) Anmeldetag: 15.02.2001
(51) Int. Cl.: B01L 3/14, C12Q 1/68

(54) **GEFÄSS ZUR NUKLEINSÄUREANALYTIK**
CONTAINER FOR NUCLEIC ACID ANALYSIS
RECIPIENT POUR L'ANALYSE D'ACIDE NUCLEIQUE

(30) Priorität: 15.02.2000 DE 10006662
(43) Veröffentlichungstag der Anmeldung: 20.11.2002
(60) Teilanmeldung: 10154591.1
(73) Patentinhaber: PreAnalytiX GmbH, 8634 Hombrechtikon (CH)
(72) Erfinder: HELFTENBEIN, Elke, 70597 Stuttgart (DE)
(74) Vertreter: Vossius & Partner
(86) Internationale Anmeldenummer: PCT/EP2001/001705
(87) Internationale Veröffentlichungsnummer: WO 2001/060517

(56) Entgegenhaltungen:
- EP-A- 0 389 063
- EP-A- 0 818 542
- WO-A-00/09746
- WO-A-97/05248
- DE-A- 19 702 907
- LOZANO ET AL: "A simple nucleic acid amplification assay for the rapid detection of Junín virus in whole blood samples" VIRUS RESEARCH, AMSTERDAM, NL, Bd. 27, 1993, Seiten 37-53, XP002900733 ISSN: 0168-1702

## Beschreibung

Die vorliegende Erfindung betrifft ein Gefäß zur Probenaufnahme, vorzugsweise zur Entnahme von Blut, wobei das entnommene Blut insbesondere zur Nukleinsäurestabilislerung, -isolierung und -analytik eingesetzt werden soll. Verfahren zur Isolierung von Nukleinsäuren sind im Stand der Technik bekannt (siehe z.B. DE 197 02 907, EP 0389 063 oder WO 97/05248).

Während der Abnahme wird Blut herkömmlicherweise in Gefäßen aufgefangen, die bereits Antikoagulantien wie z.B. Heparin, Citrat oder EDTA enthalten. So wird verhindert, daß das Blut gerinnt. So gewonnene Blutproben lassen sich längere Zeit bei geeigneten Temperaturen lagern. Diese Art der Blutgewinnung hat jedoch erhebliche Nachteile, wenn Nukleinsäuren, wie z.B. mRNA oder DNA analysiert werden sollen. Für solche Zwecke sollten die Nukleinsäuren, die in der Probe enthalten sind, am besten bereits im Moment der Abnahme stabilisiert werden, d.h. es sollte der Abbau der vorhandenen Nukleinsäuren, aber auch die Neusynthese von mRNA verhindert werden.

Dieses Ziel der stabilen Lagerung der im Probenmaterial enthaltenen Nukleinsäuren vom Moment der Abnahme an ist bei der Lagerung von Blut aus folgenden Gründen bis jetzt praktisch nicht zu bewerkstelligen:
Zellen enthalten Nukleasen, d. h. Enzyme, die Nukleinsäuren zerstören, sobald sie mit ihren Substraten (RNA, DNA) in Kontakt kommen. Die Wirkung zellulärer und extrazellulärer Nukleasen ist normalerweise unter physiologischer Kontrolle, solange die Zellen in ihrer normalen Umgebung sind. Die Entnahme von Blut führt zu mehr oder weniger starken Veränderungen der in den Zellen enthaltenen Nukleinsäuren. Nukleasen werden dann innerhalb der Zellen und/oder durch die Lyse von Zellen nach außen freigesetzt. Außerdem werden Nukleinsäuren mehr oder weniger stark synthetisiert. Gerade die Langzeitlagerung von Blut führt zur Alterung und Zerstörung der Zellen.

Ein weiteres Problem bei der Langzeitlagerung von Blutproben, die nach herkömmlichen Abnahmeverfahren gewonnen wurden, ist die starke Veränderung des Probenmaterials. Solche Veränderungen wie z.B. starke Lyse von Zellen, können dazu führen, daß die Standardverfahren der Nukleinsäureisolierung nicht mehr mit befriedigender Effizienz und Reproduzierbarkeit funktionieren.

Abgesehen von den Problemen einer stabilen Lagerung von Nukleinsäuren, die im Probenmaterial enthalten sind, ergeben sich beim herkömmlichen Verfahren der Blutentnahme weitere Schwierigkeiten. Die herkömmlichen Antikoagulantien werden oft bei der Nukleinsäureisolierung nicht mit genügender Effizienz abgetrennt und stören bei der nachfolgenden Nukleinsäureanalytik, wie z.B. bei der Amplifkation mittels PCR (Polymerase Chain Reaction). Heparin ist z.B. ein allgemein bekannter Inhibitor der PCR.

Schließlich ergibt sich bei der quantitativen Nukleinsäureanalytik die Frage, wie das gesamte Verfahren von der Probennahme bis hin zur Nukleinsäuremessung unter standardisierten Bedingungen kontrolliert werden kann. Idealerweise sollte dem Probenmaterial bereits bei der Entnahme eine in Menge und Qualität definierte Standardnukleinsäure zugesetzt werden, die dem gesamten Prozeß der Probennahme und Bestimmung unterzogen wird. Auch dies ist mit den herkömmlichen Abnahmesystemen nicht zu bewerkstelligen.

Ein weiterer Nachteil der konventionellen Blutentnahme ist die Gefahr der Übertragung von infektiösem Material, da bisher für die Nukleinsäureisolierung manuelle Verfahrensschritte notwendig sind. Ein Kontakt mit potentiell infektiösen Erregern kann nicht ausgeschlossen werden.

In der Literatur ist ein Verfahren beschrieben, bei dem die Blutprobe unmittelbar nach der Abnahme vom Patienten mit Guanidiniumsalz vermischt wird (EP 0 818 542 A1). Bei diesem Verfahren liegt das Guanidiniumsalz in Pulverform vor, um somit die höhere Stabilität des Guanidiniumsalzes zu nutzen. Dieses Verfahren besitzt jedoch gravierende Nachteile, da sich das Salz z.B. zunächst in dem zugegebenen Blut lösen muß. Der Lösungsvorgang ist insbesondere temperaturabhängig und aufgrund des verwendeten, undurchsichtigen Probenmaterials nicht zu kontrollieren. Die Verwendung eines entspechenden Produktes für diagnostisch-medizinische Zwecke ist somit äußerst problematisch.

Nukleasen sind äußerst aktive Enzyme, die nur unter extrem denaturierenden Bedingungen zu inhibieren sind. Die Denaturierung ist abhängig von der Konzentration des Guanidiniumsalzes in Lösung. Eine inhibierende Konzentration von Guanidiniumsalz in Lösung ist bei dem Verfahren der EP0818542 nicht von Anfang an gegeben. Es kommt also zum unkontrollierten Abbau von Nukleinsäuren während des Lösungsvorgangs. Bei diesem Verfahren wird außerdem auf den Zusatz von reduzierenden Agenzien verzichtet, ohne die eine wirksame Inhibition - insbesondere von RNasen - in der Regel nicht gewährleistet ist.

Die gemäß herkömmlicher Methoden erhaltene Probe kann außerdem nicht direkt für die weitere Nukleinsäureisolierung an Festphasen verwendet werden. Die Verwendung von Guanidiniumsalzpulver erlaubt außerdem nicht den Zusatz von internen Nukleinsäurestandards. Solche Standards sind zur Verfahrenskontrolle und genauen Quantifizierung jedoch unerläßlich.

Der vorliegenden Erfindung lag das technische Problem zugrunde, ein Gefäß anzugeben, das die Nachteile aus dem Stand der Technik nicht aufweist. Insbesondere soll die mit dem Gefäß entnommene Probe direkt den gängigen Nukleinsäureanalyseverfahren zugeführt werden können, ohne weitere Probenaufbereitungsschritte durchführen zu müssen.

Dieses Problem wird erfindungsgemäß gelöst durch ein Gefäß zur Probenaufnahme mit den Merkmalen des Anspruchs 1.

Weitere bevorzugte Ausführungsformen sind in den Unteransprüchen angegeben.

Das erfindungsgemäße Gefäß besitzt folgende Vorteile: 1. Die Probe, vorzugsweise Blut, wird bereits im Moment der Abnahme lysiert, indem das Abnahmegefäß bereits eine entsprechende Lyselösung, welche gleichzeitig eine Nukleinsäure-stabilisierende Lösung ist, enthält. 2. Die Nukleinsäure-stabilisierende Lösung bewirkt, daß das Probenmaterial, insbesondere die darin enthaltenen Nukleinsäuren, unmittelbar nach Kontakt mit der Lösung stabilisiert werden. 3. Die Nukleinsäure-stabilisierende Lösung ist ferner so gewählt, daß das Probenmaterial direkt in nachfolgenden Isolierungsverfahren verwendet werden kann. 4. Die Nukleinsäure-stabilisierende Lösung kann bei der nachfolgenden Isolierung so effizient abgetrennt werden, daß eine Hemmung bspw. der PCR nicht auftritt. 5. Der Nukleinsäure-stabilisierenden Lösung kann ein interner Standard zugesetzt werden. Dieser erlaubt die Kontrolle des gesamten Verfahrens von der Probenentnahme bis hin zur Nukleinsäuredetektion. 6. Die in dem Gefäß enthaltene Festphase eignet sich insbesondere für eine spätere Isolierung der daran gebundenen Nukleinsäure. Außerdem wird durch die bevorzugte Bindung der Nukleinsäuren an die Festphase die anschließende Isolierung vereinfacht, indem bereits eine erste Trennung von Nukleinsäure und weiteren Probenbestandteilen in dem Gefäß erfolgt.

Die Nukleinsäure-stabilisierende Lösung kann so gewählt werden, dass die Nukleinsäure sofort nach der Zellyse an die entsprechende Oberfläche bindet oder erst nach Zusatz weiterer Reagenzien. Der erste Fall ist bspw. gegeben wenn eine Glasoberfläche in Anwesenheit eines Guanidiniumsalzes vorgegeben wird. Der zweite Fall lässt sich zum Beispiel durch Vorlegen einer Biotin beschichteten Oberfläche und nachträglichem Hinzufügen von Streptavidin mit Nukleinsäure bindenden Eigenschaften erzielen.

Das Gefäß läßt sich grundsätzlich für die Aufnahme beliebiger Körperflüssigkeiten verwenden. Insbesondere ist es für die Aufnahme von Körperflüssigkeiten geeignet, die zelluläre Bestandteile enthalten, wie z. B. auch Knochenmark. Vorzugsweise handelt es sich jedoch um ein Gefäß zur direkten Entnahme von Vollblut aus einem Spender.

Das Gefäß besteht vorzugsweise aus einem herkömmlichen Blutentnahmegefäß (z. B. Röhrchen), in dem ein definiertes Volumen einer Nukleinsäure-stabilisierenden Lösung und eine Nukleinsäure-bindende Festphase enthalten ist. Das Röhrchen wird anschließend vorzugsweise mit einem definierten Unterdruck versehen, der ermöglicht, daß nur ein bestimmtes Volumen Blut entnommen wird. Das Röhrchen kann mit konventionellen Methoden der Blutabnahme gehandhabt werden. Die in dem Röhrchen enthaltene Lösung enthält in ihrer bevorzugten Ausführungsform folgende Reagenzien : ein Guanidiniumsalz, z. B. Guanidiniumthiocyanat, ein Detergenz, z. B. Triton-X-100, ein Reduktionsmittel, z. B. Dithiothreitol, und ein geeignetes Puffersystem wie z.B. Citrat, Tris, MES oder Hepes. In der beschriebenen Zusammensetzung ist die Lösung kompatibel mit dem Vakuumröhrchen. Die Lösung kann problemlos in dem Vakuumröhrchen gelagert werden, ohne daß es zu einer Beeinträchtigung der gewünschten stabilisierenden Funktion kommt. Das gesamte System ist insbesondere für den Blutspender problemlos und sicher bei der Probennahme.

Die Lösung, enthaltend das Guanidiniumsalz, welches als Lyse- und stabilisierende Substanz dient, die Nukleinsäure bindende Festphase, die Puffersubstanz, das Reduktionsmittel und das Detergenz ist lagerungsstabil und verwandelt das zugeführte, frisch entnommene Blut in ein Material, das ebenfalls lagerungsstabil ist und das für die weitere Nukleinsäureanalyse bzw. -isolierung unmittelbar verwendet werden kann.

Als Guanidiniumsalz sind Guanidiniumthiocyanat und/oder Guanidiniumchlorid bevorzugt.

Vorzugsweise liegt das Guanidiniumsalz in einer Konzentration von 1 bis 8,0 M vor.

Als Puffersubstanz ist Tris oder Citrat bevorzugt, wobei der exakte pH vorzugsweise mit HCl eingestellt wird. Weitere mögliche Puffer sind jedoch HEPES, MOPS, MES, Citrat- und Phosphatpuffer, wie z.B. PBS.

Als Festphase können alle Nukleinsäure bindenden Materialien eingesetzt werden. Besonders geeignet sind Glaspartikel, Nukleinsäure bindende Polymere, mit solchen beschichtete Partikel, Nukleinsäure bindende Beschichtungen des Entnahmesystem oder mit Silika beschichtete Partikel. Die Oberfläche der Nukleinsäure-bindenden Festphase kann alternativ mit spezifischen Bindemolekülen (z.B. Streptavidin, Oligonukleotide, peptide nucleic acids (PNAs), etc.) beschichtet werden, die mit Markermolekülen auf der Nukleinsäure oder mit der Nukleinsäure direkt wechselwirken. Die Formgebung der Materialien ist nur abhängig von der Form des Entnahmesystem und der nachfolgenden Isolierungsmethode. Besonders geeignet sind Formgebungen, die im Anschluß direkt bei der weiteren Verarbeitung der Nukleinsäure eingesetzt werden können, ganz besonders geeignet sind Oberflächen, die mit herkömmlichen Isolierungsverfahren kompatibel sind, wie z.B. Magnetpartikel oder Vliese.

Geeignete Festphasen sind gewerblich erhältlich, z. B. Silica beschichtete Magnetpartikel wie sie im mRNA Isolation Kit for Blood/Bone Marrow (Roche) enthalten sind.

Die Pufferkonzentration liegt vorzugsweise zwischen 10 und 300 mM, besonders bevorzugt zwischen 10 und 100 mM.

Als Detergenz ist Triton-X-100 bevorzugt. Weitere mögliche Detergenzien sind NP-40, Tween 20, Polydocanol oder andere Detergenzien.

Die Detergenzkonzentration liegt vorzugsweise bei 5 bis 30 % (w/v), besonders bevorzugt bei 10 bis 20 % (w/v).

Als Reduktionsmittel ist DTT bevorzugt, wobei jedoch auch β-Mercaptoethanol, TCEP(Tris(2-carboxyethyl)phosphin) oder andere Reduktionsmittel einsetzbar sind.

Die bevorzugte Konzentration des Reduktionsmittels liegt bei 0,1 bis 10 % (w/v); besonders bevorzugt sind 0,5 bis 2 % (w/v).

Der pH der Lösung liegt vorzugsweise bei 3,0 bis 9,0, besonders bevorzugt bei 4,0 bis 7,5.

Der pH der Lösung wird insbesondere so gewählt, daß sich nach Zugabe des Probenmaterials ein pH-Wert im Bereich von 5,0 bis 7,5 einstellt. Da durch Vorgabe des Unterdrucks sichergestellt wird, welches Probenvolumen entnommen wird, kann durch Vorlegen einer gewünschten Pufferkonzentration bzw. einem entsprechenden Volumen an Lösung gewährleistet werden, daß nach Aufnahme des vollständigen Probenvolumens der gewünschte pH auch erzielt wird. Besonders bevorzugt ist ein pH zwischen 6,3 und 6,9 nach Probenaufnahme.

Eine besonders bevorzugte Lösung enthält 4,5 M Guanidiniumthiocyanat, 50 mM Tris/HCl, 15% (w/v) Triton-X-100, 100mM DTT, eine Festphase aus Glaspartikeln oder Silika beschichteten Magnetpartikeln, wobei der pH so eingestellt wird, daß sich nach Blutzugabe ein pH von 6 bis 7,5 ergibt.

Bei einer weiteren bevorzugten Ausführungsform weist das Volumen zur Aufnahme der Blutprobe einen Unterdruck auf, der so eingestellt werden kann, das ein vorab bestimmtes Blutvolumen in das Gefäß eingesaugt wird, nachdem ein Blutgefäß angestochen wurde. Entsprechend evakuierte Gefäße sind auf dem Markt erhältlich.

Das Gefäß, enthaltend das entnommene Blut, kann dann sofort der weiteren Analytik zugeführt werden oder aber für einen längeren Zeitraum (bis mehrere Tage oder Wochen) ohne Nachteile für die Qualität der Probe aufbewahrt werden.

Bei dem erfindungsgemäßen Verfahren wird das frisch entnommene Blut direkt in dem Blutentnahmegefäß mit der oben beschriebenen Lösung in Kontakt gebracht, so daß sofort sämtliche Vorgänge, die das Nukleinsäuremuster der Probe verändern können, gestoppt werden. Die Nukleinsäuren können vorzugsweise bereits im Gefäß an der Festphase gebunden vorliegen oder in einem weiteren Reaktionsschritt an die Festphase gebunden werden.

Die später im Rahmen der Nukleinsäureanalytik ermittelten Daten hinsichtlich der nachgewiesenen Nukleinsäuren stellen daher sehr genau den Ist-Zustand zum Zeitpunkt der Blutentnahme, dar, sowohl hinsichtlich der Mengen als auch der Arten der Nukleinsäuren.

Vorzugsweise entspricht die entnommene Blutmenge dem 0,1- bis 4-fachen der in dem Gefäß vorgelegten Lösung. Letztere beträgt vorzugsweise 0,5 bis 5,0 ml. Somit liegt die Endkonzentration an Guanidiniumsalz nach Blutzusatz vorzugsweise bei 1,0 bis 5 M, vorzugsweise bei 1,0 bis 3,0 M.

Das erfindungsgemäße Gefäß wird vorzugsweise dann zur Blutentnahme eingesetzt, wenn die Blutprobe für die Nukleinsäureanalytik verwendet werden soll.

Die Verwendung o.g. Lösung als Bestandteil des beschriebenen Abnahmesystems garantiert allein die sofortige Lyse der Zellen und simultane Stabilisierung der Probe durch unmittelbare Inaktivierung der Nukleasen. Überraschenderweise kann die so gewonnene Blutprobe selbst bei Raumtemperatur über mehrere Tage gelagert werden. Das Abnahmesystem gewährleistet außerdem eine kontaminationssichere und nichtinfektiöse Handhabung von der Probennahme über die Nukleinsäureisolierung bis hin zur Analytik. Bei den herkömmlichen Verfahren der Nukleinsäureisolierung sind bisher immer zusätzliche Handhabungsschritte (wie die Überführung der entnommenen Blutprobe in die Reagenzien zur Nukleinsäureisolierung usw.) notwendig, die mit einem zusätzlichen Infektionsrisiko oder Kontaminationsrisiko der Probe verbunden sind.

Überraschenderweise kann die an die Festphase gebundene Nukleinsäure auch nach längerer Lagerung einfach aus dem Probenmaterial isoliert werden. Die Anwesenheit der Festphase während der Probenlyse führt zu einer sofortigen Bindung der Nukleinsäuren an die Oberfläche. Hierdurch werden Ausbeuteverluste, verursacht z.B. durch Präzipitatbildung, die nach längerer Lagerung auftreten können, verhindert, da die Oberfläche mit der darin parktisch quantitativ gebundenen Nukleinsäure einfach aus dem System entfernt werden kann.

Die mit dem Blutentnahmesystem gewonnene Probe kann gängigen Nukleinsäureisolierungsverfahren zugeführt werden; bei Verwendung von Silika beschichteten Magnetpartikeln ist es möglich auf gängige Standardverfahren der Nukleinsäureisolierung zurückzugreifen (Magnetseparation, Waschen, Elution der Nukleinsäure).

Die vorliegende Erfindung besteht somit aus einem Probenaufnahmesystem, das so konzipiert ist, daß folgende Bedingungen erfüllt werden: 1. Kontrollierte Probenentnahme und gleichzeitige Stabilisierung der im Probenmaterial enthaltenen Nukleinsäuren (DNA, RNA). 2. Probenentnahme, bei der auf den Einsatz von Antikoagulantien vollkommen verzichtet werden kann. 3. Bindung der Nukleinsäuren (unmittelbar oder nach weiterem Verfahrenschritt) an eine im System enthaltene Festphase. 4. Die mit dem beschriebenen System gewonnene Probe kann leicht in bestehende Nukleinsäureisolierungssysteme integriert werden. 5. Das System samt darin enthaltener Probe ist lagerungsstabil.

Zusätzlich wurde überraschenderweise festgestellt, daß die mit dem beschriebenen Abnahmesystem gewonnene Probe in dem Gefäß über längere Zeit ohne Degradation der Nukleinsäuren lagerungsstabil ist.

Die folgenden Beispiele erläutern die Erfindung.

### Abb. 1:

Probenentnahmegefäß mit Nukleinsäure-stabilisierender Substanz (N-sS), definiertem Vacuum, mit Festphase versetzt und mit Septum versiegelt.

### Abb. 2:

Graphische Darstellung einer Gelanalyse (1 % Agarose) von 28S und 18S rRNA, die im Probeentnahmegefäß unterschiedlich lange gelagert wurde. Spalte 1: Isolierung und Auftrennung der RNA unmittelbar nach Probenentnahme (keine Lagerung), Spalte 2: Lagerung für einen Monat bei -20°C, Spalte 3: Lagerung für 6 Tage bei 4°C. Die Menge der aufgetragenen RNA entsprach einem Blutvolumen von 120 µl.

### Abb 3:

Graphische Darstellung einer Gelanalyse (1 % Agarose) von DNA, die im Probeentnahmegefäß unterschiedlich lange gelagert wurde. Spalte 1: Isolierung unmittelbar nach Probenentnahme (keine Lagerung), Spalte 2: Lagerung für einen Monat bei -20°C, Spalte 3: Lagerung für 6 Tage bei 4°C. Die Menge der aufgetragenen DNA entsprach einem Blutvolumen von 10 µl.

### Abb. 4:

Graphische Darstellung einer Gelanalyse von isolierter MS2-RNA nach Inkubation in Serum/Stabilisierungslösung mit/ohne DTT nach 180 min bei 40 °C. Spalte 1: Positiv Kontrolle: MS-2 RNA; Spalte 2: DNA Marker; Spalten 3 bis 5: MS-2 RNA nach Inkubation mit DTT-haltiger Stabilisierungslösung (3-fach Bestimmung); Spalten 6 bis 8: MS-2 RNA nach Inkubation mit Stabilisierungslösung ohne DTT (3-fach Bestimmung).

### Abb. 5:

Graphische Darstellung einer Gelanalyse von MS2-RNA, die nach Inkubation in Serum / Stabilisierungslösung für 3 Tage bei 40°C isoliert wurde. Der Guanidiniumthiocyanatgehalt (GTC Gehalt) der Stabilisierungslösung nach Serumzugabe, in welcher die betreffende RNA inkubiert wurde, ist in der entsprechenden Spalte angegeben.

Spalte 1: 2,70M GTC, Spalte 2: 2,5 M GTC, Spalte 3: 2,36 M GTC, Spalte 4: 2,2M GTC, Spalte 5: 2,08 M GTC, Spalte 6: 1,94 M GTC, Spalte 7: 1,80 M GTC, Spalte 8: 1,66M GTC.

### Abb. 6:

Graphische Darstellung einer Gelanalyse der PCR Amplifikate von MS2-RNA, welche nach 1 bzw. 8 Tagen Inkubation bei 40°C in Serum/Stabilisierungslösung isoliert wurde.
Spalte 1: Amplifikat der nach einem Tag isolierten RNA, Spalte 2: Amplifikat der nach 8 Tagen isolierten RNA. Spalte 3: DNA Marker, Spalte 4: MS2-RNA positiv Kontrolle: 0,8µg in 10µl RT 1:50 verdünnt, 1µl amplifiziert.

### Abb. 7:

Graphische Darstellung einer Gelanalyse von isolierter MS2-RNA nach 6 (Spalten 2-12) bzw. 13 (Spalten 14-19) Tagen Inkubation bei Raumtemperatur in Serum/Stabilisierungslösung. Hinter den betreffenden Spalten steht der pH-Wert, welcher nach Mischung von Serum und Stabilisierungslösung erreicht wurde.

Spalte 1, 13, 20: DNA-Marker, Spalte 2: pH 8.0, Spalte 3: pH 7.7, Spalte 4: pH 7.5, Spalte 5: pH 7.35, Spalte 6: pH 7.18, Spalten 7, 14: pH 7.07, Spalten 8, 15: pH 6.94, Spalten 9, 16: pH 6.8, Spalten 10, 17: pH 6.72, Spalten 11, 18: pH 6.68, Spalten 12, 19: pH 6.7. Die Stabilisierungslösung der RNA in Spalten 12, 19 hatten den gleichen pH Wert, wie die der RNA in Spalte 11, enthielt aber 5M GTC anstelle von 4 M.

### Abb. 8:

Graphische Darstellung des Nachweises von RNA und DNA im Standard Agarosegel (1 % Agarose). Spalte 1: Molekulargewichtsmarker, Spalte 2 bis 4: Isolierte Nukleinsäuren; Spalte 2: Nukleinsäure aus Vollblutlysat versetzt mit MS2 RNA (7 Tage), Spalte 3: Nukleinsäure: aus Vollblutlysat versetzt mit MS2 RNA (0 Tage, Kontrolle), Spalte 4: Nukleinsäure aus Vollblutlysat (7 Tage), Spalte 5: Nukleinsäure aus Vollblutlysat (0 Tage, Kontrolle). Die oberen Banden zeigen chromosomale DNA (bei allen 4 Proben deutlich erkennbar), die unteren Banden in den Spalten 2 und 3 zeigen die zugesetzte und isolierte MS2 RNA.

### Beispiel 1:

### Blutentnahmesystem

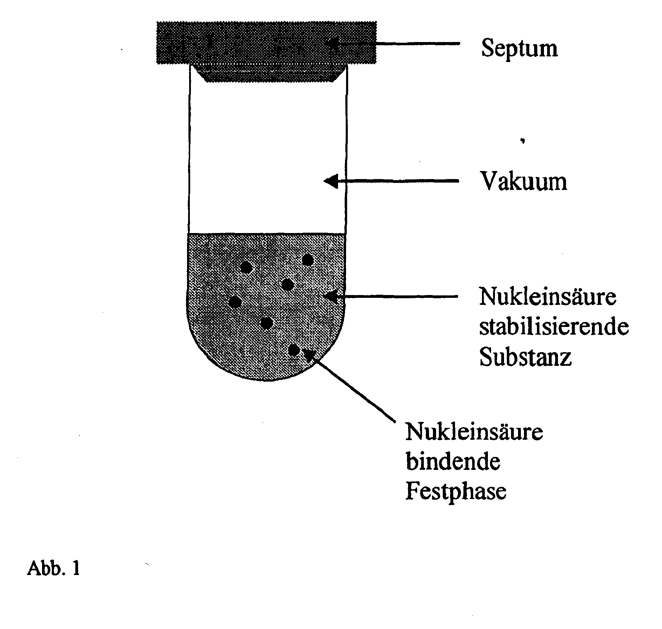

Das Blutentnahmesystem kann in einer bevorzugten Ausführungsform folgendermaßen zusammengesetzt sein (s. Abb. 1): Ein Röhrchen wird mit einem definierten Volumen der Nukleinsäure-stabilisierenden Lösung gefüllt, mit einer Nukleinsäure-bindenden Festphase und mit einem definierten Vakuum versehen, dann mit einem Septum verschlossen. Das Septum ist so konstruiert, daß es mit dem gängigen Probenentnahmezubehör (Kanüle usw.) kompatibel ist. Im vorliegenden Beispiel wurden 2,2 ml Reagenz vorgelegt und das Vakuum war so eingestellt, daß bei der Probennahme exakt 2,2 ml Blut zufließen konnten. Die im zufließenden Blutstrom enthaltenen Nukleinsäuren wurden unmittelbar in eine stabile Form überführt.

Allgemeine Vorbemerkung zu den nachfolgenden Beispielen.

Wenn nicht anders erwähnt hatte bei allen nachfolgend beschriebenen Beispielen die Nukleinsäure-stabilisierende Substanz (N-sS) folgende Zusammensetzung: 45 mM Tris, 5 M Guanidiniumthiocyanat, 0,8% (w/v) Dithiothreitol, 18 % (w/v) Triton-X-100, pH 6,0.

In allen beschriebenen Beispielen wurde die Nukleinsäure-stabilisierende Substanz mit der Probe im Verhältnis 1 zu 1 gemischt (1 Volumen N-sS plus 1 Volumen Probenmaterial).

Für alle Beispiele wurde Blut dadurch stabilisiert, daß es unmittelbar bei der Entnahme in das mit N-sS versetzte Röhrchen gegeben wurde.

### Beispiel 2:

### Stabilität von Nukleinsäure nach Mischung von Probenmaterial und N-sS.

Isolierung von RNA und DNA vom Probenlysat mit silikaderivatisierten Oberflächen.

### Material und Methode:

Das Probenmaterial für die DNA- und RNA-Isolierung wurde unmittelbar nach der Entnahme, nach Lagerung für 6 Tage bei 4°C und nach Lagerung für 1 Monat bei -20°C verwendet.

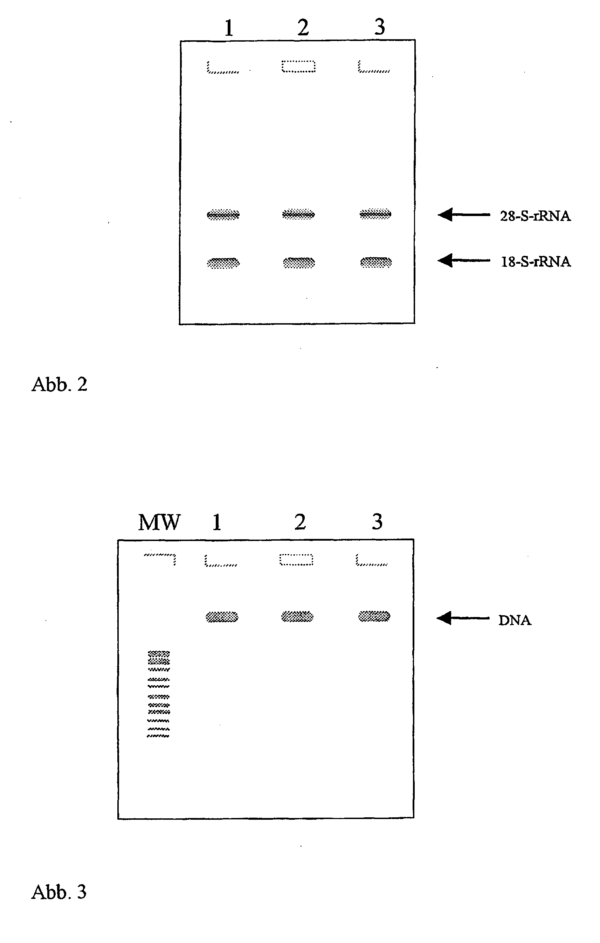

Für die Isolierung von RNA (Abb. 2) wurde der HighPure RNA Isolation Kit (Boehringer Mannheim, Kat.-Nr. 1828 665) verwendet. Die Beipackzettelvorschrift wurde folgendermaßen modifiziert: Ein Volumen von 2,4 ml Probenlysat wurde in 4 Aliquots mit jeweils 600 µl auf die Säule aufgetragen, so daß insgesamt Probenmaterial aus 2,4 ml Lysat aufgetragen wurden. Alle anderen Schritte wurden entsprechend dem Beipackzettel ausgeführt. Die RNA wurde schließlich mit 100 µl Elutionspuffer eluiert.

Zur Isolierung von DNA (Abb. 3) wurde der QiaAmp Blood Kit (Qiagen-Kat.-Nr. 29104) eingesetzt. Die im Beipackzettel beschriebene Standardprozedur wurde in verschiedenen Punkten modifiziert: 400 µl Probenvolumen wurden direkt auf die Säule gegeben, wobei das im Kit enthaltene Bindereagenz nicht eingesetzt wurde. 25 µl Proteinase-K-Stocklösung wurden zugefügt und die Probe 10 Min. bei Raumtemperatur inkubiert. Danach wurde die Säule in ein Sammelgefäß gestellt und wie im Beipackzettel beschrieben zentrifugiert. Alle weiteren Schritte wurden, bis auf die Verwendung von Ethanol, wie im Beipackzettel beschrieben durchgeführt. Das Elutionsvolumen war 200 µl.

### Beispiel 3

### Bedeutung von reduzierenden Reagenzien (z.B. DTT) in der Stabilisierungslösung für die Langzeitstabilisierung von RNA Material und Methode:

### Verwendete Stabilisierungslösung:

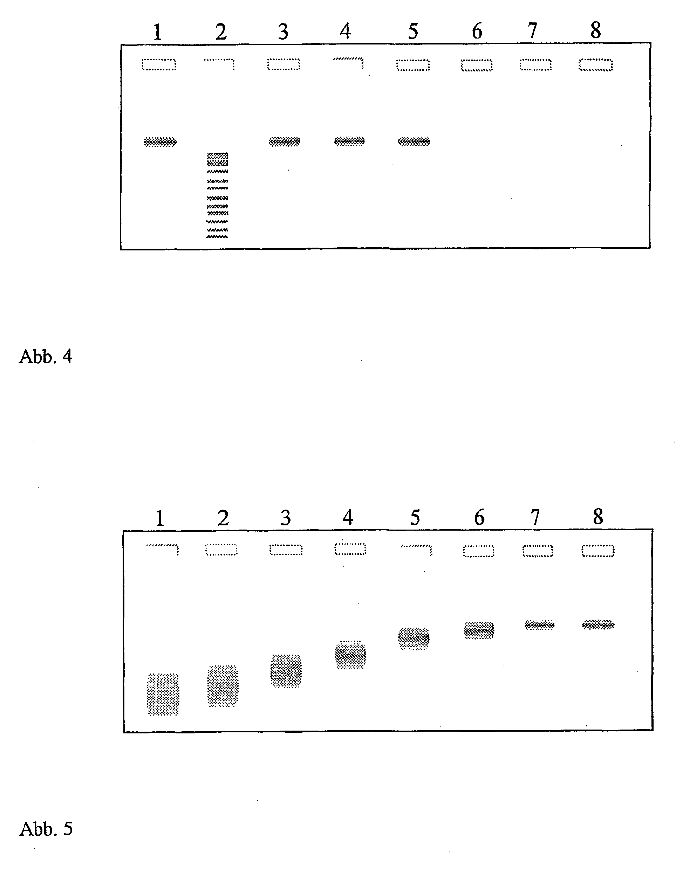

4.0 M GTC; 13.5 % Triton X100; 45 mM Tris/HCl; mit bzw. ohne 120 mM DTT. 700 µl Serum wurden mit 700 µl Stabilisierungslösung gemischt. Nach 2 min Inkubation wurden 20 µl MS2-RNA (0.8 µg/µl von Roche Diagnostics) zugegeben. Die Proben wurden für 180 min bei 40 °C inkubiert und anschließend in Aliquots a 400 µl mit dem High Pure total RNA Kit von Roche entsprechend Experiment 1 aufgearbeitet. Die Proben wurden in 50 µl eluiert und bei - 20 °C eingefroren. Die Analytik erfolgte mittels Agarosegel (siehe Abb. 4).

Ergebnis: Ohne den Zusatz von reduzierenden Reagenzien zur Stabilisierungslösung kann keine Langzeitstabilisierung von RNA erreicht werden.

### Beispiel 4

### Stabilität von MS2-RNA in Serum/Stabilisierungslösung: Abhängigkeit von der GTC Konzentration

### Material und Methode

Verwendete Stabilisierungslösungen: 3 - 5 M GTC; 13.5 % Triton X100; 50 mM DTT; 42 mM Tris/HCl;
pH der Lösungen: ca. 4.0;
pH der Lösungen nach Serumzugabe: ca. 6.7.

2 ml Serum wurden mit 2.5 ml der jeweiligen Stabilisierungslösungen gemischt. Nach einer Inkubationszeit von 2-5 min wurden 90 µl MS2-RNA (0.8 µg/µl von Roche) zugegeben und bei 40 °C inkubiert. In regelmäßigen Abständen wurden 400 µl Probe entnommen und mit dem High Pure total RNA Kit von Roche entsprechend Experiment 1 aufgearbeitet. Die Proben wurden in 50 µl eluiert und bei - 20 °C eingefroren. Für die Analyse der RNA-Integrität wurden 20 µl des Eluates auf ein 1,5 %iges Agarosegel aufgetragen (Abb. 5). Die RT-PCR-Analytik erfolgte mittels AMV-RT und PCR. Jeweils 10 µl der Eluate wurden mittels AMV-RT (Roche) reverse transkribiert und anschließend mittels quantitativer PCR auf dem Lightcycler analysiert.

| | | |
|---|---|---|
| Ansatz für RT: (42 °C für 1 h) | 4.0 µl | AMV-RT-Puffer |
| | 2.0 µl | dNTP's (Endkonzentration 10 mM) |
| | 0.5 µl | RNaseinhibitor (Roche, 20 units) |
| | 1.0 µl | Primer 2827 (Endkonzentration 1 µM) |
| | 1.9 µl | DMPC-Wasser |
| | 0.6 µl | AMV-RT (Roche, 15 units) |
| | 10 µl | Template-RNA |
| | 20 µl | |

Die PCR wurde auf dem Lightcycler bei einer Annealingtemperatur von 61 °C unter Verwendung von SYBR-Green als Detektionssystem durchgeführt. Alle Proben mit einem Treshold-Cycle größer 20 werden als negativ betrachtet, da das detektierte Signal ausschießlich auf die Bildung von Primerdimeren zurückzuführen ist. Dies kann durch die Analyse der Schmelzkurven am LightCycler (Roche) eindeutig bewiesen werden. Das RT-Produkt wurde 1:50 mit bidest. Wasser verdünnt und 1 µl davon für eine 10 µl-PCR gemäß folgendem Schema eingesetzt:

| | | |
|---|---|---|
| Ansatz für PCR: | 1.6 µl | MgCl₂ (Stammlsg. 25 mM) |
| | 5.9 µl | DMPC-Wasser |
| | 0.25 µl | Primer 2827 (Stammlsg. 20 mM) |
| | 0.25 µl | Primer 2335 (Stammlsg. 20 mM) |
| | 1.0 µl | SYBR-Green-Mastermix (Roche) |
| | 1.0 µl | RT-Ansatz |
| | 10 µl | |

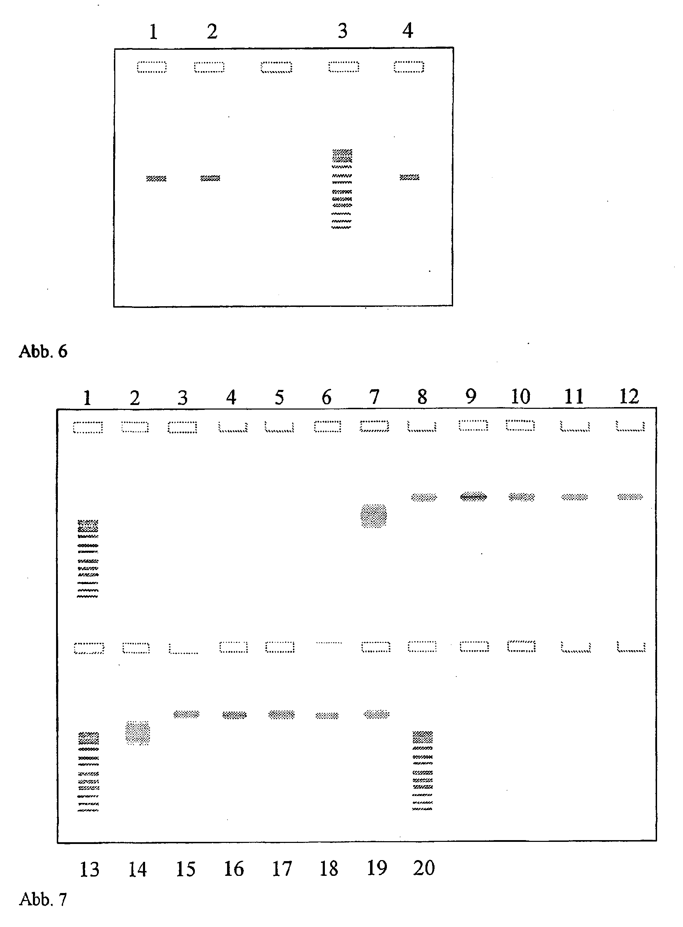

Das Amplifikat der PCR wurde vollständig auf ein 2 %iges Agarosegel aufgetragen (siehe Abb. 6).

### Ergebnis:

Abb. 5 zeigt die eluierte MS2-RNA nach 3 Tagen Inkubation bei 40 °C detektiert im Agarosegel. Obwohl nach 8 Tagen bei 40 °C noch alle RNA-Proben amplifiziert und eindeutig nachgewiesen werden können, sind bereits nach 3 Tagen deutliche Unterschiede der RNA-Integrität in Abhängigkeit vom GTC-Gehalt zu erkennen. Demnach ist ein Salzgehalt kleiner 2 M in der Serum/Stabilisierungslösung für die Integrität der RNA von Vorteil.

Nicht gezeigt ist die Tatsache, daß MS2-RNA bereits 2 min nach Zusatz zu Serum vollständig von RNasen abgebaut wird und damit keine RNA mehr nachweisbar ist. Mit diesem Beispiel konnte belegt werden, daß der Abbau der RNA durch Zusatz von Stabilisierungslösung zum Serum deutlich verzögert werden kann. Nach 8 Tagen bei 40 °C in Serum/Stabilisierungslösung kann MS2-RNA mittels PCR ohne Probleme detektiert werden (Abb 6).

### Beispiel 5

### Stabilität von MS2-RNA in Serum/Stabilisierungslösung: Abhängigkeit vom pH-Wert der mit Stabilisierungslösung versetzten Probe.

### Material und Methode

| | | |
|---|---|---|
| Verwendete Lösung: | 4 M (5 M) | GTC |
| | 14,4% | Triton X 100 |
| | 50 mM | DTT |
| | 45 mM | Tris HCl |
| pH nach Serumzugabe zwischen 6,7 und 8,0 | | |

2,5 ml Stabilisierungslösung wurden mit 2,0 ml Serum gemischt. Nach Zugabe von 90 µl MS2 RNA (0,8 µg/ml, Roche) wurden die Proben bei Raumtemperatur inkubiert. In regelmäßigen Abständen wurde die RNA aus 500 µl Probe mit dem Roche viral RNA Kit entsprechend Beispiel 4 aufgearbeitet und in 50µl Elutionspuffer isoliert. 20µl des Eluates wurden mittels Agarosegel analysiert (siehe Abb. 7).

### Ergebnis:

Der pH der Serum 1 Stabilisierungslösung und damit auch der pH und Pufferbereich der Stabilisierungslösung ist für die Langzeitstabilisierung von RNA entscheidend. Während bei einem pH Wert von 8,0 bereits nach 2 Tagen keine intakte RNA mehr nachgewiesen werden konnte, ist in einem pH Bereich zwischen 6,6 und 7,0 noch nach 13 Tagen Inkubation bei Raumtemperatur intakte RNA nachweisbar. Neben dem pH Wert ist jedoch auch eine optimal eingestellte GTC Konzentration für die Langzeitstabilisierung von RNA von Bedeutung (siehe Beispiel 4). Das dargestellte Beispiel verdeutlicht, daß für eine Langzeitstabilisierung von RNA eine GTC Endkonzentration in der stabilisierten Probe von 2,2 M GTC besser ist als 2,8 M.

### Beispiel 6

### Stabilität einer Nukleinsäure bindenden Oberfläche in Gegenwart von Stabilisierungslösung gezeigt unter Verwendung von Silika beschichteten Magnetpartikeln.

### Material und Methode

| | | |
|---|---|---|
| Verwendete Lösung: | 4,5 M | GTC |
| | 15 % | Triton-X-100 |
| | 100 mM | DTT |
| | 50 mM | MES |

Die Silika beschichteten Magnetpartikel wurden dem mRNA Isolation Kit for Blood/Bone Marrow (Roche Molecular Biochemicals) entnommen. Die verwendete Menge Partikel pro ml betrug ca. 35 mg. Das Blutentnahmesystem, bestehend aus dem Entnahmeröhrchen, der Stabilisierungslösung und den Magnetpartikeln, wurde 14 Tage bei Raumtemperatur gelagert. Anschließend wurde mit diesem System Vollblut entnommen. Als Kontrolle wurde ein frisch hergestelltes Entnahmesystem (Röhrchen, Stabilisierungslösung, Magnetpartikel) verwendet. Aus beiden Ansätzen erfolgte die Isolierung der im Probenmaterial enthaltenen Nukleinsäuren. Die Magnetpartikel wurden mit einem Magneten abgetrennt, der Überstand wurde verworfen. Die Partikel wurden in 50% Ethanol, 10mM Tris, pH 7,0 resuspendiert und mehrmals mit derselben Lösung gewaschen. Schließlich wurden die Partikel in 10mM Tris/HCl pH 7,0 auf 70°C erhitzt, wobei sich die Nukleinsäure von den Magnetpartikeln löst. Die Partikel wurden magnetisch separiert und der Nukleinsäure enthaltende Überstand im Standard Agarosegel analysiert.

### Ergebnis:

**Tabelle 1:**

| | | |
|---|---|---|
| Nach 14 Tagen Lagerung hat sich die Nukleinsäure bindende Eigenschaft der Festphase nicht verändert. Die Probe, als auch die Kontrolle zeigen dieselben Nukleinsäure bindenden Eigenschaften. | | |

| | Probe (14 Tage, RT) | Kontrolle (0 Tage) |
|---|---|---|
| Nukleinsäure im Gel nachweisbar | + | + |

### Beispiel 7

### Stabilität, Isolierung und Nachweis von DNA und RNA, nach 7 Tagen Lagerung bei gleichzeitiger Bindung an Silika beschichtete Magnetpartikel.

### Material und Methode

| | | |
|---|---|---|
| Verwendete Suspension: | 4,5 M | GTC |
| | 15 % | Triton-X-100 |
| | 100 mM | DTT |
| | 50 mM | MES |
| | 35 mg/ml | Partikel |

4 Blutentnahmesysteme (Röhrchen) enthaltend 1 ml der oben beschriebenen Suspension wurde mit 1 ml Vollblut versetzt. Zwei der Röhrchen (Vollblutlysat) wurde zusätzlich mit 25µg MS2 RNA versetzt. Je ein Röhrchen der beiden Ansätze (Vollblutlysat +/- MS2 RNA) wurde unmittelbar danach zur Nukleinsäureisolierung eingesetzt (Durchführung siehe Beispiel 6). Die beiden anderen Röhrchen wurden 7 Tage bei Raumtemperatur gelagert: Nach diesem Zeitraum wurde die Nukleinsäureisolierung durchgeführt. Das Elutionsvolumen betrug 200µl pro 200µl Vollblutvolumen. Die Nukleinsäuren wurden im Standard Agarosegel analysiert.

### Ergebnis:

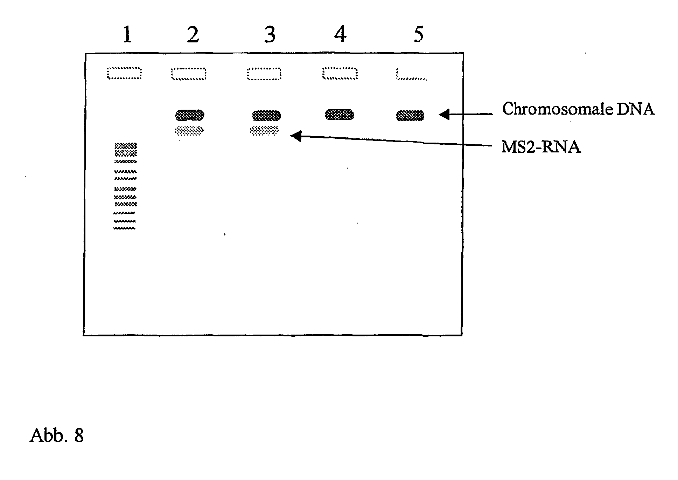

Nach 7 Tagen Lagerung im Probennahmesystem (Lösung, Festphase) ist die Stabilität von chromosomaler DNA und MS2-RNA nachweislich gegeben (Abb.8).

## Patentansprüche

1. Gefäß zur Probenaufnahme, vorzugsweise Blutaufnahme, enthaltend eine wässrige Lösung, die ein Guanidiniumsalz, eine Puffersubstanz, ein Reduktionsmittel und ein Detergenz enthält und die Nukleinsäuren stabilisiert, und eine Festphase, die Nukleinsäuren binden kann, **dadurch gekennzeichnet, dass** es einen Unterdruck im zur Probenaufnahme vorgesehenen Raum aufweist.

2. Gefäß nach Anspruch 1, **dadurch gekennzeichnet, dass** das Guanidiniumsalz ausgewählt wird aus Guanidiniumthiocyanat und Guanidiniumchlorid.

3. Gefäß nach einem der Ansprüche 1 oder 2, **dadurch gekennzeichnet, dass** das Guanidiniumsalz in einer Konzentration von 1 bis 8 M vorliegt.

4. Gefäß nach einem der Ansprüche 1 bis 3, dadurch gekennzeichet, dass die Lösung nach Zugabe von Probenmaterial einen pH-Wert von 4 bis 7,5 aufweist, und die Puffersubstanz ausgewählt wird aus Tris(hydroxymethyl)-aminomethan (Tris), 2-(4-(2-Hydroxyethyl)- 1-piperazinyl)-ethansulfonsäure (HEPES), 3-(N-Morpholino)-Propansulfonsäure (MOPS), 2-(N-Morpholino)ethansulfonsäure (MES), Citrat und Phosphatpuffer.

5. Gefäß nach einem der Ansprüche 1 bis 4, **dadurch gekennzeichnet, dass** die Festphase getrennt als Vlies, Filter, Partikel, Gel, Kugel, Zapfen und/oder Stab vorliegt und/oder direkt mit dem Gefäß verbunden ist.

6. Gefäß nach einem der Ansprüche 1 bis 5, **dadurch gekennzeichnet, dass** die Puffersubstanz in einer Konzentration von 10 bis 300 mM vorliegt.

7. Gefäß nach einem der Ansprüche 1 bis 6, **dadurch gekennzeichnet, dass** das Detergenz ausgewählt wird aus Triton-X-100, NP-40, Polydocanol und Tween 20.

8. Gefäß nach einem der Ansprüche 1 bis 7, **dadurch gekennzeichnet, dass** das Detergenz in einer Konzentration von 5 bis 30 Gew. % vorliegt.

9. Gefäß nach einem der Ansprüche 1 bis 8, **dadurch gekennzeichnet, dass** das Reduktionsmittel ausgewählt wird aus Dithiothreitol (DTT), β-Mercaptoethanol und Tris(2-chlorethyl)phosphat (TCEP).

10. Gefäß nach einem der Ansprüche 1 bis 9, **dadurch gekennzeichnet, dass** das Reduktionsmittel in einer Konzentration von 0,1 bis 10,0 Gew. % vorliegt

11. Gefäß nach einem der Ansprüche 1 bis 10, **dadurch gekennzeichnet, dass** der pH der Lösung zwischen 4,0 und 7,5 liegt.

12. Gefäß nach einem der Ansprüche 1 bis 11, **dadurch gekennzeichnet, dass** nach Zugabe des Probenmaterials ein pH-Wert zwischen 5,0 und 7,5 erhalten wird.

13. Gefäß nach einem der Ansprüche 1 bis 12, **dadurch gekennzeichnet, dass** die Lösung folgende Bestandteile enthält: 4,5 M Guanidiniumthiocyanat; 50 mM MES; 15 % (w/v) Triton-X-100; 100 mM DTT, wobei der pH so eingestellt ist, dass nach Blutzugabe ein pH von 6,0 bis 7,5 vorliegt.

14. Verfahren zum Stabilisieren und/oder Isolieren von Nukleinsäure aus Vollblut, umfassend den Schritt des Einbringens von Vollblut in ein Gefäß nach einem der Ansprüche 1 bis 13 und gegebenenfalls Isolieren von festphasengebundener Nukleinsäure.

## Claims

1. A receptacle for receiving samples, preferably for receiving blood, containing an aqueous solution that comprises a guanidinium salt, a buffer substance, a reducing agent and a detergent, and that stabilises nucleic acids, and a solid phase capable of binding nucleic acids, **characterised in that** it has an underpressure in the space intended for sample uptake.

2. The receptacle according to claim 1, **characterised in that** the guanidinium salt is selected from guanidinium thiocyanate and guanidinium chloride.

3. The receptacle according to claim 1 or 2, **characterised in that** the guanidinium salt is present at a concentration of 1 to 8 M.

4. The receptacle according to any one of claims 1 to 3, **characterised in that** after addition of sample material, the solution has a pH value of 4 to 7.5 and the buffer substance is selected from Tris(hydroxymethyl)aminomethane (Tris), 2-(4-(2-hydroxyethyl)-1-piperazinyl)-ethanesulphonic acid (HEPES), 3-(N-morpholino)-propanesulphonic acid (MOPS), 2-(N-morpholino)ethanesulphonic acid (MES), citrate and phosphate buffer.

5. The receptacle according to any one of claims 1 to 4, **characterised in that** the solid phase is present separately as fleece, filter, particle, gel, ball, plug and/or rod and/or is directly connected with the receptacle.

6. The receptacle according to any one of claims 1 to 5, **characterised in that** the buffer substance is present in a concentration of 10 to 300 mM.

7. The receptacle according to any one of claims 1 to 6, **characterised in that** the detergent is selected from Triton-X-100, NP-40, Polydocanol and Tween 20.

8. The receptacle according to any one of claims 1 to 7, **characterised in that** the detergent is present in a concentration of 5 to 30 weight %.

9. The receptacle according to any one of claims 1 to 8, **characterised in that** the reducing agent is selected from dithiothreitol (DTT), beta-mercaptoethanol and Tris(2-chlorethyl)phosphate (TCEP).

10. The receptacle according to any one of claims 1 to 9, **characterised in that** the reducing agent is present in a concentration of 0.1 to 10.0 weight %.

11. The receptacle according to any one of claims 1 to 10, **characterised in that** the pH value of the solution is between 4.0 and 7.5.

12. The receptacle according to any one of claims 1 to 11, **characterised in that** after addition of the sample material a pH value of between 5.0 and 7.5 is obtained.

13. The receptacle according to any one of claims 1 to 12, **characterised in that** the solution has the following components: 4.5 M guanidinium thiocyanate; 50 mM MES; 15% (w/v) Triton-X-100; 100 mM DTT, wherein the pH is adjusted so that a pH of 6.0 to 7.5 is present after the addition of blood.

14. A method of stabilising and/or isolating nucleic acid from whole blood, comprising the step of introducing the whole blood into a receptacle according to any one of claims 1 to 13 and, optionally, isolating solid-phase-bound nucleic acid.

## Revendications

1. Récipient pour la réception d'échantillons, de manière préférée pour la réception de sang, contenant une solution aqueuse, qui contient un sel de guanidine, une substance tampon, un agent réducteur et un détergent et qui stabilise des acides nucléiques, et une phase solide, qui peut lier des acides nucléiques, **caractérisé en ce que** ledit récipient présente une sous-pression dans l'espace prévu pour la réception d'échantillons.

2. Récipient selon la revendication 1, **caractérisé en ce que** le sel de guanidine est choisi parmi du thiocyanate de guanidine et du chlorure de guanidine.

3. Récipient selon la revendication 1 ou 2, **caractérisé en ce que** le sel de guanidine est présent en une concentration de 1 à 8 M.

4. Récipient selon l'une quelconque des revendications 1 à 3, **caractérisé en ce que** la solution présente une valeur pH de 4 à 7,5 après ajout du matériau d'échantillon, et la substance tampon est choisie parmi le tris(hydroxyméthyl-aminométhane (Tris), l'acide 2-(4-(2-hydroxyéthyl)-1-pipérazinyl)-éthanesulfonique (HEPES), l'acide 3-(N-morpholino)-propanesulfonique (MOPS), l'acide 2-(N-morpholino)éthanesulfonique (MES), des tampons citrate et phosphate.

5. Récipient selon l'une quelconque des revendications 1 à 4, **caractérisé en ce que** la phase solide est présente de manière séparée en tant que nappe, filtre, particule, gel, bille, bâtonnet et/ou barrette et/ou est reliée directement au récipient.

6. Récipient selon l'une quelconque des revendications 1 à 5, **caractérisé en ce que** la substance tampon est présente en une concentration de 10 à 300 mM.

7. Récipient selon l'une quelconque des revendications 1 à 6, **caractérisé en ce que** le détergent est choisi parmi Triton-X-100, NP-40, polydocanol et Tween 20.

8. Récipient selon l'une quelconque des revendications 1 à 7, **caractérisé en ce que** le détergent est présent en une concentration de 5 à 30 % en poids.

9. Récipient selon l'une quelconque des revendications 1 à 8, **caractérisé en ce que** l'agent de réduction est choisi parmi le dithiothréitol (DTT), le β-mercaptoéthanol et le tris(2-choréthyl) phosphate (TCEP).

10. Récipient selon l'une quelconque des revendications 1 à 9, **caractérisé en ce que** l'agent réducteur est présent en une concentration de 0,1 à 10,0 % en poids.

11. Récipient selon l'une quelconque des revendications 1 à 10, **caractérisé en ce que** le pH de la solution se situe entre 4,0 et 7,5.

12. Récipient selon l'une quelconque des revendications 1 à 11, **caractérisé en ce qu'**une valeur pH comprise entre 5,0 et 7,5 est obtenue après ajout du matériau d'échantillon.

13. Récipient selon l'une quelconque des revendications 1 à 12, **caractérisé en ce que** la solution contient les composants suivants : 4,5 M de thiocyanate de guanidine ; 50 mM de MES; 15 % (poids/volume) de Triton-X-100 ; 100 mM de DTT, le pH étant ajusté de manière à ce qu'un pH de 6,0 à 7,5 soit présent après aj out du sang.

14. Procédé de stabilisation et/ou d'isolation d'acide nucléique à partir de sang total, comprenant l'étape consistant à introduire du sang total dans un récipient selon l'une quelconque des revendications 1 à 13 et éventuellement l'étape consistant à isoler des acides nucléiques liés à une phase solide.
